# EUROPÄISCHE PATENTSCHRIFT

(11) **EP 1 504 113 B1**
(45) Veröffentlichungstag und Bekanntmachung des Hinweises auf die Patenterteilung: **15.11.2006**
(21) Anmeldenummer: 03730061.3
(22) Anmeldetag: 16.05.2003
(51) Int. Cl.: C12Q 1/00, G01N 33/543

(54) **VERFAHREN ZUR HERSTELLUNG VON POLYMERSCHICHTEN**
METHOD FOR PRODUCING POLYMER LAYERS
PROCEDE DE FABRICATION DE COUCHES POLYMERES

(30) Priorität: 16.05.2002 DE 10221840; 16.05.2002 DE 10221845; 16.05.2002 DE 10221846
(43) Veröffentlichungstag der Anmeldung: 09.02.2005
(73) Patentinhaber: F. HOFFMANN-LA ROCHE AG, 4070 Basel (CH); Roche Diagnostics GmbH, 68305 Mannheim (DE)
(72) Erfinder: HORN, Carina, 64665 Alsbach-Haehnlein (DE); HOENES, Joachim, 64673 Zwingenberg (DE); KNAPPE, Wolfgang-Reinhold, 67071 Ludwigshafen (DE)
(74) Vertreter: Weiss, Wolfgang
(86) Internationale Anmeldenummer: PCT/EP2003/005179
(87) Internationale Veröffentlichungsnummer: WO 2003/097859

(56) Entgegenhaltungen:
- EP-A- 0 691 408
- WO-A-02/052045
- US-A- 5 407 818
- US-A- 5 693 034
- US-A- 5 863 650

## Beschreibung

Die Erfindung betrifft ein Verfahren zur Herstellung von Polymerschichten auf einem Träger durch Photopolymerisation einer polymerisierbaren flüssigen Zusammensetzung, eine zur Herstellung von Polymerschichten auf einem Träger geeignete Vorrichtung und ein Verfahren zur Herstellung eines Sensors, der eine Polymerschicht mit einem darin eingelagerten Indikator enthält.

Die Herstellung von Polymerschichten auf Trägern ist bekannt. Hierzu wird eine polymerisierbare Flüssigkeit in einer möglichst gleichmäßigen Dicke auf den Träger aufgebracht und vollständig polymerisiert. Die Polymerisation kann durch chemische Polymerisationsinitiatoren oder durch Bestrahlung der Flüssigkeit gestartet werden.

Im Zusammenhang mit der Herstellung von Klebstofffilmen sind Polymerisationstechniken bekannt, bei denen eine Mischung von funktionalisierten. Acrylatpräpolymeren auf einen Träger aufgebracht werden und durch Bestrahlung von der Oberfläche der Polymermasse ganz oder teilweise durchgehärtet werden. Die Steuerung der Schichtdicke der Polymerisation erfolgt durch Wahl der Belichtungsintensität und -dauer und durch Eigenabsorption der eingesetzten Präpolymere.

Nachteil bei den Verfahren des Standes der Technik ist, dass ein oftmals hoher Aufwand zur Einstellung einer vorbestimmten und gleichmäßigen Dicke der Polymerschicht auf dem Träger erforderlich ist. Weiterhin findet man bei konventionellen Polymerisationsmethoden oftmals nur eine geringe Haftung der Polymerschicht auf dem Träger.

Die der vorliegenden Erfindung zugrunde liegende Aufgabe bestand darin, die geschilderten Nachteile des Standes der Technik mindestens teilweise zu vermeiden. Insbesondere sollte ein Verfahren zur Herstellung von Polymerschichten auf einem Träger bereitgestellt werden, welches auf einfache Weise die Herstellung von Polymerschichten mit einer vorbestimmten und gleichmäßigen Schichtdicke ermöglicht.

Im Gegensatz zu den im Stand der Technik im Zusammenhang mit der Klebstoffherstellung beschriebenen Verfahren, bei denen geschlossene, hydrophobe Polymerschichten erzeugt werden, sollen offene, zur Aufnahme von wässriger Probenflüssigkeit und Analyt befähigte, hydrophile Schichten bereitgestellt werden.

Diese Aufgabe wird dadurch gelöst, dass eine flüssige polymerisierbare Zusammensetzung auf einen Träger aufgebracht wird und dort nicht vollständig, sondern nur in einer Schicht mit vorbestimmter Dicke unmittelbar auf dem Träger polymerisiert wird. Auf diese Weise können gleichmäßige und sehr gut am Träger haftende Schichten auf einfache Weise hergestellt werden.

Ein Gegenstand der Erfindung ist somit ein Verfahren zur Herstellung von Polymerschichten auf einem transparenten Träger, umfassend die Schritte:
(a) Bereitstellen eines Trägers,
(b) Aufbringen einer photopolymerisierbaren flüssigen Zusammensetzung auf den Träger,
(c) Bestrahlen der photopolymerisierbaren flüssigen Zusammensetzung durch den Träger derart, dass eine nur teilweise Polymerisation der flüssigen Zusammensetzung erfolgt und eine Polymerschicht mit einer vorbestimmten Dicke auf dem Träger gebildet wird, und
(d) Entfernen der verbleibenden flüssigen Zusammensetzung von der Polymerschicht.

Der zur Herstellung von Polymerschichten verwendete Träger ist ein zumindest teilweise optisch transparenter Träger, beispielsweise ein Kunststoffträger, wie etwa Polycarbonatfolie, Celluloseacetatfolie, Polyesterfolie oder Polyetherfolie, ein Glasträger oder ein Quarzträger. Auch Träger aus Verbundmaterialien können eingesetzt werden. Bei Verwendung eines solchen zumindest teilweise optisch transparenten Trägers erfolgt die Bestrahlung der flüssigen Zusammensetzung vorzugsweise durch den Träger hindurch. Die Dicke des Trägers wird günstigerweise so gewählt, dass einerseits eine ausreichende mechanische Stabilität für die Polymerschicht und andererseits eine ausreichende Durchlässigkeit für das zur Bestrahlung verwendete Licht vorgesehen wird. Beispielsweise verwendet man Träger mit einer Dicke von 5 µm bis 20 mm. Ebenfalls bevorzugt ist die Verwendung von UV-transparenten Trägern.

Die photopolymerisierbare flüssige Zusammensetzung enthält zumindest eine photopolymerisierbare Substanz, d.h. eine durch Bestrahlung (ggf. in Anwesenheit eines Photoinitiators) polymerisierbare Substanz. Bevorzugte Beispiele für solche Substanzen sind photopolymerisierbare Monomere, wie z.B. olefinisch ungesättigte Substanzen, d.h. Substanzen mit einer C=C-Bindung. Weitere Beispiele für geeignete photopolymerisierbare Substanzen sind funktionalisierte Oligo- oder Polymere, die unter Bestrahlung mit Licht quervernetzen. Solche photoaktiven Funktionalisierungen umfassen z.B. Acrylate, Azide, Carbazide, Sulfonazide, Diazoketone, Dimethylmaleinimide, photozyklisierbare Reste (z.B. Chalkone) und Benzophenderivate. Geeignete Oligo- oder Polymere sind z.B. Polyurethane, Polyvinylalkohole, Polyester, Polyether, Polyvinylpyrrolidone, Polyacrylate oder Oligosaccharide. Besonders bevorzugt werden die photopolymerisierbaren Substanzen ausgewählt aus acrylischen Monomeren, wie etwa Acrylamid, Acrylestern, z.B. Polyethylenglycoldiacrylat, vinylaromatischen Monomeren, wie etwa 4-Vinylbenzolsulfonsäure, funktionalisierten Polyvinylpyrrolidonen und beliebigen Kombinationen von einer oder mehrerer der genannten Substanzen.

In einer bevorzugten Ausführungsform wird eine wässrige polymerisierbare Zusammensetzung verwendet, in der die zu polymerisierenden Monomere in gelöster Form vorliegen. Zweckmäßigerweise werden daher für diese Ausführungsform hydrophile Monomere eingesetzt, die eine ausreichend hohe Löslichkeit in einem wässrigen Lösungsmittel besitzen.

Die Initiation der Polymerisation in der flüssigen Zusammensetzung wird durch Bestrahlen mit Licht bewirkt. Vorzugsweise enthält die Zusammensetzung einen oder mehrere Photoinitiatoren, um die Polymerisation auszulösen. Beispiele für geeignete Photoinitiatoren sind radikalische Initiatoren, wie Benzophenone, Benzile, Anthrachinone, Thiosulfonsäuren, Azoverbindungen, oder ionische Initiatioren, wie z.B. Triarylsulfoniumsalze, Aryliumhexafluoroantimonate.

Die Bestrahlung der Zusammensetzung erfolgt durch den Träger hindurch, so dass die Polymerisation an der Trägeroberfläche beginnt und - je nach Einstellung der Polymerisationsbedingungen - in einer vorbestimmten Entfernung über der Trägeroberfläche aufgrund der Lichtabsorption innerhalb der polymerisierbaren Flüssigkeit endet. Verwerfungen oder Abweichungen von der horizontalen Lage beim Träger können die Dicke der Polymerschicht nicht beeinflussen. Mechanische Toleranzen werden weitgehend beseitigt.

Die Dicke der Polymerschicht kann durch geeignete Maßnahmen in einem breiten Bereich eingestellt werden. Insbesondere kann die Schichtdicke des Polymers durch Variation der Bestrahlungsintensität, der Bestrahlungsdauer oder/und den Zusatz von Polymerisationsinhibitoren, z.B. UVabsorbierenden Substanzen zur flüssigen Zusammensetzung gesteuert werden. Weiterhin ist eine Steuerung auch durch die Trägerdicke oder/und das Trägermaterial möglich. Die Dicke der Polymerschichten beträgt vorzugsweise ≤ 500 µm und besonders bevorzugt ≤ 100 µm. Bei der Anwendung der Polymerschichten als Sensoren, insbesondere als Biosensoren, werden oftmals auch geringere Schichtdicken von vorzugsweise ≤ 50 µm, insbesondere von ≤ 5 µm, hergestellt.

Wenn die Polymerschicht als Bestandteil eines Sensors eingesetzt werden soll, enthält sie zweckmäßigerweise neben den bereits genannten Bestandteilen einen Indikator, z.B. einen optischen oder/und elektrochemischen Indikator, der auf Parameter in dem die Polymerschicht umgebenden Medium reagieren kann. Der Indikator kann der photopolymerisierbaren flüssigen Zusammensetzung zugesetzt oder - insbesondere wenn es sich um ein kleineres Molekül handelt - in die bereits fertige Polymerschicht eindiffundiert werden. Wenn die Polymerschicht mehrere Indikatoren enthalten soll, sind auch Kombinationen der zuvor genannten Maßnahmen möglich.

Der Indikator kann auch ein Makromolekül sein, beispielsweise mit einem Molekulargewicht ≥ 10 kD und insbesondere ≥20 kD sein. Besonders bevorzugt verwendet man als Indikator eine katalytische Substanz, beispielsweise ein Enzym, das gegebenenfalls in Form eines Enzym-Coenzym-Komplexes vorliegen kann. Besonders bevorzugte Beispiele für Enzyme sind Oxidoreduktasen, insbesondere Dehydrogenasen, wie etwa Glucose-Dehydrogenase (E.C.1.1.1.47) oder Oxidasen. Coenzyme sind vorzugsweise organische Moleküle, die kovalent oder nicht-kovalent an eine Enzym gebunden sind und durch die Umsetzung mit einem Substrat des Enzyms verändert, beispielsweise oxidiert oder reduziert werden. Bevorzugte Beispiele für Coenzyme sind Flavin-, Nicotin-, Chinonderivate, wie etwa FAD, FADH₂, NAD⁺, NADH/H⁺, NADP⁺, NADPH/H⁺ oder PQQ.

Die Polymerschicht kann neben Enyzmen und gegebenenfalls Coenzymen auch Mediatoren enthalten, d.h. Substanzen, die in der Lage sind, eine Regeneration von Coenzymen zu bewirken. In diesem Fall wirkt das Enzym als katalytischer Indikator, d.h. es kann mehrere Moleküle eines Substrats, beispielsweise eines in einer zugegebenen Probe vorhandenen Analyten, wie etwa Glucose im Blut, umsetzen.

In einer besonders bevorzugten Ausführungsform enthält die Polymerschicht einen Enzym-Coenzym-Komplex als stöchiometrischen Reaktionspartner für ein nachzuweisendes Enzymsubstrat. Hier wird das Coenzym einmalig umgesetzt und nicht regeneriert. In dieser Ausführungsform ist die Verwendung von Mediatoren, verbunden mit dem Einsatz komplexer Reagenzgemische geringer Stabilität und hoher Störungsanfälligkeit, nicht mehr erforderlich.

Bei Verwendung von makromolekularen Substanzen als Indikatoren kann durch geeignete Vernetzung des Polymers (z.B. durch Verwendung bioder/und polyfunktionellen Monomeren) eine vernetzte Polymerschicht hergestellt werden, in der die makromolekulare Indikatorsubstanz in immobilisierter Form eingelagert ist, während niedermolekulare Substanzen, wie etwa Coenzym, Enzymsubstrat etc. in die Schicht eindiffundieren können.

Die Herstellung der Polymerschicht kann als kontinuierlicher Prozess durchgeführt werden, wobei eine kontinuierliche Bildung einer Polymerschicht in einer auf einen Träger aufgebrachten flüssigen photopolymerisierbaren Zusammensetzung bewirkt wird. Bei der kontinuierlichen Polymerisation wird vorzugsweise die photopolymerisierbare flüssige Zusammensetzung kontinuierlich an einer ersten Position auf einen sich bewegenden Träger aufgebracht und kontinuierlich an einer zweiten Position bestrahlt. Selbstverständlich kann auch der Träger stationär gehalten werden und die Positionen des Flüssigkeitsauftrags und der Bestrahlung bewegt werden. Ebenso sind diskontinuierliche Methoden zur Herstellung der Polymerfilme denkbar. Diesen Ausführungsformen gemeinsam ist, dass aufgrund der Bestrahlung der photopolymerisierbaren flüssigen Zusammensetzung durch den Träger hindurch, eine unmittelbar am Träger beginnende und nicht vollständige Polymerisation der Flüssigkeit erfolgen kann.

Noch ein weiterer Aspekt der Erfindung ist eine Vorrichtung zur Herstellung von Polymerschichten, umfassend
(a) Mittel zum Aufnehmen und gegebenenfalls zum Transport eines Trägers,
(b) Mittel zum Aufbringen einer photopolymerisierbaren flüssigen Zusammensetzung auf den Träger und
(c) Mittel zum Bestrahlen der photopolymerisierbaren flüssigen Zusammensetzung durch den Träger hindurch, um die Bildung einer Polymerschicht mit vorbestimmter Dicke auf dem Träger zu bewirken, und
(d) gegebenenfalls Mittel zum Entfernen von nicht polymerisierter flüssiger Zusammensetzung von der Polymerschicht.

Das Verfahren und die Vorrichtung können zur Herstellung eines Sensors verwendet werden, wobei in die Polymerschicht ein Indikator, beispielsweise ein Biomolekül, wie etwa ein Enzym, eingelagert wird. Der Indikator kann in der Polymerschicht in immobilisierter Form vorliegen. Besonders bevorzugt ist der Indikator ein Enzym, gegebenenfalls in Form eines Enzym-Coenzym-Komplexes. Der Sensor kann beispielsweise ein optischer oder/und elektrochemischer Sensor sein. Besonders bevorzugt ist der Sensor ein fluoreszenzbasierender Sensor.

Noch ein weiterer Gegenstand der Erfindung ist ein Verfahren zur Herstellung eines Sensors, umfassend die Schritte:
(a) Bereitstellen eines Trägers,
(b) Aufbringen einer photopolymerisierbaren flüssigen Zusammensetzung, die mindestens einen Indikator enthält, auf den Träger,
(c) Bestrahlen der photopolymerisierbaren flüssigen Zusammensetzung durch den Träger derart, dass eine Polymerschicht mit einer vorbestimmten Dicke auf dem Träger gebildet wird,
(d) Entfernen der verbleibenden flüssigen Zusammensetzung von der Polymerschicht und
(e) Einbringen des Trägers mit der den Indikator enthaltenden Polymerschicht in einen Sensor, der Mittel zum Nachweis der Reaktion des Indikators mit einem Analyten in einer Probe enthält.

Die Nachweismittel sind insbesondere optische oder/und elektrochemische Nachweismittel. Besonders bevorzugt sind die Nachweismittel optische Nachweismittel, umfassend eine Lichtquelle zur Bestrahlung der Polymerschicht und einen Detektor zum Auffangen von Licht aus der Polymerschicht. Die Lichtquelle, z.B. ein Laser oder eine LED, ist vorzugsweise zum Einstrahlen von Licht durch den Träger in die Polymerschicht vorgesehen. Der Detektor ist vorzugsweise zum Auffangen von Lichtstrahlung, z.B. von Fluoreszenzemission, aus der Polymerschicht vorgesehen.

Der Sensor kann zur Bestimmung beliebiger Analyten, beispielsweise physikalisch-chemischer Parameter, wie etwa Temperatur, Partialdrücke von Gasen, wie etwa O₂, CO₂, NOₓ etc., oder zur Bestimmung biochemischer Parameter, wie etwa Analyten in biologischen Proben, z.B. Körperflüssigkeiten, verwendet werden.

Weiterhin soll die Erfindung durch die nachfolgenden Abbildungen und Beispiele erläutert werden.

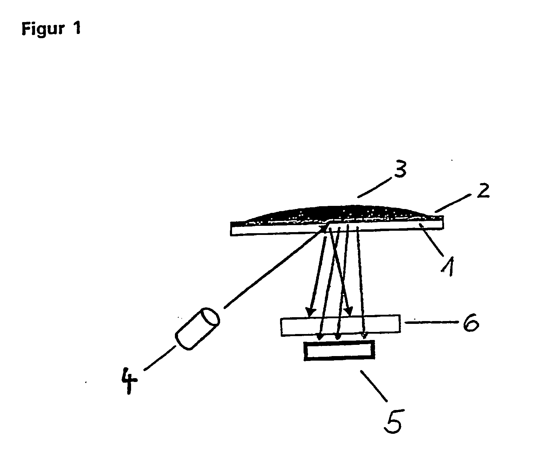

**Figur 1** zeigt eine erste Ausführungsform eines durch das erfindungsgemäße Verfahren hergestellten Sensors. Auf einem optisch transparenten Träger (1) ist eine Polymerschicht (2), mit einem Indikator, z.B. mit einem Nachweisreagenz für eine enzymatische Reaktion, aufgebracht. Auf die Polymerschicht wird eine Probe (3), z.B. Blut, gegeben. Die Bestimmung der enzymatischen Reaktion zwischen dem in der Probe (3) enthaltenen Analyten und dem in der Polymerschicht (2) enthaltenen Nachweisreagenz erfolgt durch optische Methoden. Licht aus einer Lichtquelle (4), z.B. einem Laser oder einer LED, wird von unten (durch den Träger) auf die Reagenzschicht (2) eingestrahlt. Von der Probe zurück gestrahltes Absorptions- oder Fluoreszenzlicht wird in einem Detektor (5) nachgewiesen. Gegebenenfalls wird - insbesondere zum Nachweis von Fluoreszenzlicht - ein optisches Filterelement (6) vor den Detektor geschaltet, um ein Übersprechen des Fluoreszenzanregungslichts zu blockieren.

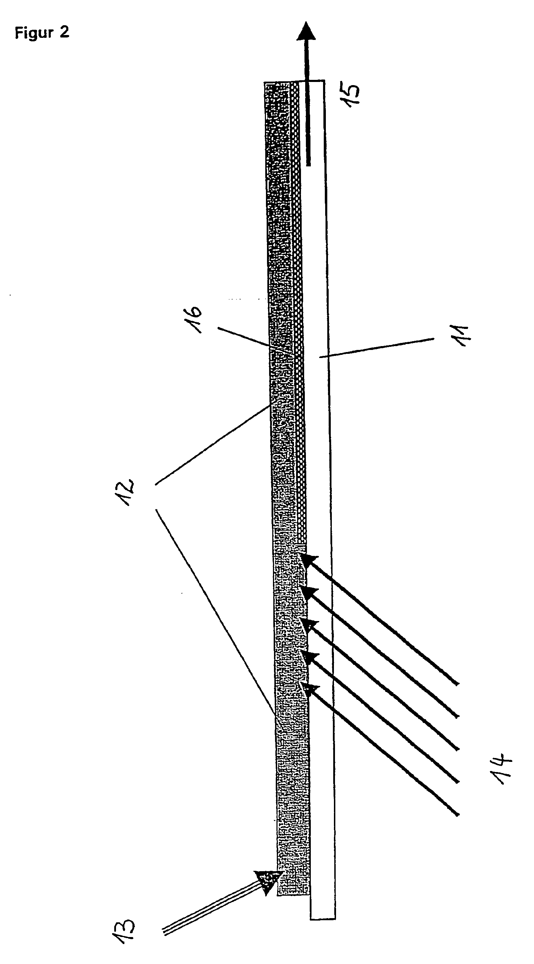

**Figur 2** zeigt die Herstellung einer erfindungsgemäßen Polymerschicht. Auf einen optisch transparenten Träger (11), z.B. einer Plastikfolie, wird ein flüssiges Reagenz (12) beispielsweise an einer ersten Position (13) aufgebracht. Das flüssige Reagenz (12) wird an einer zweiten Position von unten durch den Träger (11) hindurch mit Licht aus einer Lichtquelle (14) bestrahlt. Gleichzeitig wird der Träger in die mit Pfeil gekennzeichnete Richtung (15) bewegt. Es wird unmittelbar auf dem Träger (11) eine polymerisierte Reagenzschicht (16) gebildet. Über der Polymerschicht (16) befindet sich überschüssiges flüssiges Reagenz. Die Dicke der polymerisierten Reagenzschicht (16) kann durch die Reagenzzusammensetzung, die Dauer und Intensität der Lichteinstrahlung sowie durch die Eigenschaften des Trägers (11) gesteuert werden.

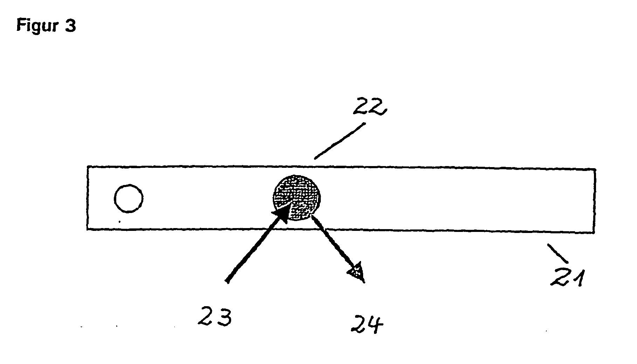

**Figur 3** zeigt eine Ausführungsform eines Fluoreszenz-basierenden Sensors von unten. Eine, beispielsweise durch das kontinuierliche Verfahren in Figur 2 hergestellte Polymerschicht, die einen Indikator enthält, kann geschnitten und unter Verwendung bekannter Techniken in einen Sensor (21) eingebracht werden. Nach Aufbringen der Probe auf die Oberseite wird aus einer Lichtquelle von unten Anregungslicht (23), z.B. UV-Licht, eingestrahlt. Die durch die Reaktion des Analyten mit dem Nachweisreagenz in der Polymerschicht (22) erzeugte Fluoreszenz (24), z.B. blaues Licht, wird mit einem Detektor nachgewiesen.

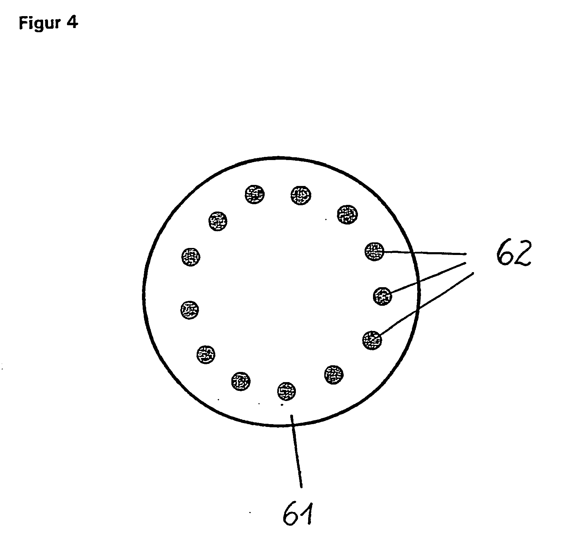

Auch mehrere (gleiche oder verschiedene) Reagenzien können auf einem Träger aufgebracht werden. Ein Beispiel für eine solche Ausführungsform in Form einer Scheibe ist in Figur 4 gezeigt. Auf dem optisch transparenten Träger (31) sind mehrere aus Polymerschichten mit Indikatoren bestehende Reagenzspots (32) angeordnet.

### Beispiele

### Beispiel 1: Nachweis von Glucose in dem System Glucose-Dehydrogenase (GlucDH)/NAD⁺ in einem Polymerfilm

Eine Suspension folgender Substanzen wurde in einem Plastikreagenzglas vermischt.

### Rezeptur 1

| **Substanz** | **Menge [g]** | **Gew -%** |
|---|---|---|
| Acrylamid | 2,5 | 22,02 |
| Methylenbisacrylamid | 0,7 | 6,17 |
| 2,2-Dimethoxy-2-phenylacetophenon | 0,05 | 0,44 |
| Glycerin | 5 | 44,05 |
| Hydroxyethylmethacrylat | 1,4 | 12,33 |
| Methylmethacrylat | 0,4 | 3,52 |
| Crodasinic O-Lsg., pH8, 0,3 g/1000 ml | 1 | 8,81 |
| N,N'-(1,2-Dihydroxyethylen)bisacrylamid | 0,3 | 2,64 |
| **SUMME** | **11,35** | **100** |

0,5 ml dieser Suspension wurden mit 0,5 ml einer Lösung von GlucDH (100 mg/ml) versetzt und die Mischung im Ultraschallbad luftblasenfrei homogenisiert.

Die klare Lösung wurde auf eine coronabehandelte Folie gegossen und 20 min mit einer herkömmlichen Belichtungsapparatur (Isel-UV-Belichtungsgerät 2) durch den Träger hindurch belichtet. Die Folie wurde kurz mit Wasser gewaschen und anschließend an der Luft getrocknet.

Die sich ergebende Schichtdicke war < 2 µm. Eine frisch angesetzte Glucose/NAD⁺-Lösung (GKL-3-Lösung, 300 mg/dl Glucose, 1 ml/6,4 mg NAD⁺) wurde auf den Film aufgetüpfelt. Sofort war unter der UV-Lampe eine starke Fluoreszenz sichtbar.

### Beispiel 2: Beeinflussung der Schichtdicke durch Zugabe eines UV-Adsorbers

Es wurde eine Polymerschicht hergestellt, die einen blauen Farbstoff (Absorptionsmaximum ≈ 650 nm) zur besseren Erkennung enthielt (Rezeptur 2). In einem weiteren Versuch wurde der Ausgangsrezeptur ein gelber Farbstoff als UV-Absorber beigemischt (Rezeptur 3).

### Rezeptur 2

| **Substanz** | **Menge** | **Gew -%** |
|---|---|---|
| Acrylamid | 37,5 g (0,53 mol) | 25,78 |
| Polyethylenglycol-diacrylat, Mw ≈ 575 g/mol | 52,5 g (ca. 0,96 mol) | 36,10 |
| Lösung von Crodasinic O (0,3 g/1 l) | 50 g | 34,38 |
| 4-Vinylbenzolsulfonsäure | 5 g | 3,44 |
| Photoinitiator 2,2-Dimethoxy-2-phenylacetophenon | 350 mg | 0,24 |
| New Methylenblau N | 100 mg | 0,06 |
| **SUMME** | **145,45 g** | **100** |

Die Mischung wurde durch Rühren und durch Ultraschallbad-Behandlung homogenisiert, auf eine 140 *µ*m Pokalonfolie (coronabehandelt, Stufe 4) mit der Pipette verteilt und auf einem UV-Belichtungsgerät (Actina U4, W. Lemmen GmbH) 1 min belichtet.

Die sich ergebende Schichtdicke wurde mit einer Mikrometerschraube gemessen und betrug 240,5 µm.

### Rezeptur 3

| **Substanz** | **Menge** | **Gew.[%]** |
|---|---|---|
| Rezeptur 2 | 1 ml | ca. 99,99 |
| Mordant Yellow 7 (Nr. 686) (UV-Absorber) | 0,0001 g | 0,001 |
| **SUMME** | **ca. 1,0001 g** | **100** |

Die Mischung wurde, wie zuvor beschrieben, auf einer Folie verteilt und dann polymerisiert. Die sich ergebende Schichtdicke wurde mit einer Mikrometerschraube gemessen und betrug 79,3 µm.

Dieses Experiment zeigt, dass sich eine Schichtdickenbeeinflussung realisieren lässt. Ohne UV-Absorber beträgt die Schichtdicke bei ansonsten gleichen Reaktionsbedingungen 240,5 *µ*m (siehe oben); mit UV-Absorber (Mordant Yellow 7) lediglich 79,3 *µ*m.

## Patentansprüche

1. Verfahren zur Herstellung von Polymerschichten auf einem transparenten Träger, umfassend die Schritte:
(a) Bereitstellen eines Trägers,
(b) Aufbringen einer photopolymerisierbaren flüssigen Zusammensetzung auf den Träger,
(c) Bestrahlen der photopolymerisierbaren flüssigen Zusammensetzung durch den Träger derart, dass eine Polymerschicht mit einer vorbestimmten Dicke auf dem Träger gebildet wird, und
(d) Entfernen der verbleibenden flüssigen Zusammensetzung von der Polymerschicht.

2. Verfahren nach Anspruch 1,
**dadurch gekennzeichnet,**
**dass** man einen zumindest teilweise optisch transparenten Träger verwendet.

3. Verfahren nach Anspruch 1 oder 2,
**dadurch gekennzeichnet,**
**dass** man einen Träger mit einer Dicke von mindestens 5 *µ*m verwendet.

4. Verfahren nach einem der vorhergehenden Ansprüche,
**dadurch gekennzeichnet,**
**dass** man einen Träger, ausgewählt aus einem Kunststoffträger, einem Glasträger oder einem Quarzträger, verwendet.

5. Verfahren nach einem der vorhergehenden Ansprüche,
**dadurch gekennzeichnet,**
**dass** man eine photopolymerisierbare flüssige Zusammensetzung verwendet, die zumindest eine photopolymerisierbare Substanz enthält.

6. Verfahren nach Anspruch 5,
**dadurch gekennzeichnet,**
**dass** man Monomere, ausgewählt aus acrylischen Monomeren, vinylaromatischen Monomeren, funktionalisierten Polyvinylpyrrolidonen und beliebigen Kombinationen davon, verwendet.

7. Verfahren nach einem der vorhergehenden Ansprüche,
**dadurch gekennzeichnet,**
**dass** man eine photopolymerisierbare flüssige Zusammensetzung verwendet, die zumindest einen Photoinitiator enthält.

8. Verfahren nach einem der vorhergehenden Ansprüche,
**dadurch gekennzeichnet,**
**dass** man die Schichtdicke des Polymers durch Variation von
(i) der Bestrahlungsintensität,
(ii) der Bestrahlungsdauer oder/und
(iii) den Zusatz von Substanzen, die das Polymerisationslicht absorbieren,
steuert.

9. Verfahren nach einem der vorhergehenden Ansprüche,
**dadurch gekennzeichnet,**
**dass** man eine Polymerschicht mit einer Dicke von ≤ 500 *µ*m, insbesondere von ≤ 5 *µ*m, herstellt.

10. Verfahren nach einem der vorhergehenden Ansprüche,
**dadurch gekennzeichnet,**
**dass** man eine Polymerschicht herstellt, die zumindest einen Indikator enthält.

11. Verfahren nach Anspruch 10,
**dadurch gekennzeichnet,**
**dass** man als Indikator eine makromolekulare Substanz verwendet.

12. Verfahren nach Anspruch 10 oder 11,
**dadurch gekennzeichnet,**
**dass** man als Indikator eine katalytische Substanz, insbesondere ein Enzym, verwendet.

13. Verfahren nach einem der vorhergehenden Ansprüche,
**dadurch gekennzeichnet,**
**dass** man eine vernetzte Polymerschicht herstellt.

14. Verfahren nach Anspruch 13,
**dadurch gekennzeichnet,**
**dass** man ein Makromolekül in die vernetzte Polymerschicht einschließt.

15. Verfahren nach einem der vorhergehenden Ansprüche,
**dadurch gekennzeichnet,**
**dass** man das Bestrahlen mit UV-Licht durchführt.

16. Verfahren nach einem der vorhergehenden Ansprüche,
**dadurch gekennzeichnet,**
**dass** man die Polymerisation als kontinuierlichen Prozess durchführt.

17. Verfahren nach Anspruch 16,
**dadurch gekennzeichnet,**
**dass** die photopolymerisierbare flüssige Zusammensetzung kontinuierlich an einer ersten Position auf einen sich bewegenden Träger aufgebracht wird und kontinuierlich an einer zweiten Position bestrahlt wird.

18. Vorrichtung zur Herstellung von Polymerschichten, umfassend:
(a) Mittel zum Aufnehmen und gegebenenfalls zum Transport eines Trägers,
(b) Mittel zum Aufbringen einer photopolymerisierbaren flüssigen Zusammensetzung auf den Träger und
(c) Mittel zum Bestrahlen der photopolymerisierbaren flüssigen Zusammensetzung durch den Träger hindurch, um die Bildung einer Polymerschicht mit vorbestimmter Dicke auf dem Träger zu bewirken, und
(d) gegebenenfalls Mittel zum Entfernen von nicht polymerisierter flüssiger Zusammensetzung von der Polymerschicht.

19. Verwendung des Verfahrens nach einem der Ansprüche 1 bis 17 oder der Vorrichtung nach Anspruch 18 zur Herstellung eines Sensors, wobei die Polymerschicht zumindest einen Indikator enthält.

20. Verwendung nach Anspruch 19,
**dadurch gekennzeichnet,**
**dass** der Indikator in der Polymerschicht in immobilisierter Form vorliegt.

21. Verwendung nach Anspruch 19 oder 20,
**dadurch gekennzeichnet,**
**dass** die Polymerschicht ein Enzym, gegebenenfalls in Form eines Enzym-Coenzym-Komplexes, enthält.

22. Verwendung nach einem der Ansprüche 19 bis 21,
**dadurch gekennzeichnet,**
**dass** der Sensor aus optischen oder/und elektrochemischen Sensoren ausgewählt wird.

23. Verfahren zur Herstellung eines Sensors, umfassend die Schritte:
(a) Bereitstellen eines transparenten Trägers,
(b) Aufbringen einer photopolymerisierbaren flüssigen Zusammensetzung, die mindestens einen Indikator enthält, auf den Träger,
(c) Bestrahlen der photopolymerisierbaren flüssigen Zusammensetzung durch den Träger derart, dass eine Polymerschicht mit einer vorbestimmten Dicke auf dem Träger gebildet wird,
(d) Entfernen der verbleibenden flüssigen Zusammensetzung von der Polymerschicht
(e) Einbringen des Trägers mit der den Indikator enthaltenden Polymerschicht in einen Sensor, der Mittel zum Nachweis der Reaktion des Indikators mit einem Analyten in einer Probe.

24. Verfahren nach Anspruch 23,
**dadurch gekennzeichnet,**
**dass** der Indikator ein Enzym, gegebenfalls in Form eines Enzym-Coenzym-Komplexes, umfasst.

25. Verfahren nach Anspruch 24,
**dadurch gekennzeichnet,**
**dass** die Nachweismittel optische oder/und elektrochemische Nachweismittel umfassen.

26. Verfahren nach Anspruch 25,
**dadurch gekennzeichnet,**
**dass** die optischen Nachweismittel eine Lichtquelle zur Bestrahlung der Polymerschicht und einen Detektor zum Auffangen von Licht aus der Polymerschicht umfassen.

27. Verfahren nach Anspruch 26,
**dadurch gekennzeichnet,**
**dass** die Lichtquelle zum Einstrahlen von Licht durch den Träger hindurch in die Polymerschicht vorgesehen wird.

28. Verfahren nach Anspruch 26 oder 27,
**dadurch gekennzeichnet,**
**dass** ein Detektor zum Auffangen von Fluoreszenzemission aus der Polymerschicht vorgesehen wird.

## Claims

1. Method for producing polymer layers on a transparent support, comprising the steps:
(a) provision of a support,
(b) application of a photopolymerizable liquid composition to the support,
(c) irradiation of the photopolymerizable liquid composition through the support in such a way that a polymer layer with a predetermined thickness is formed on the support, and
(d) removal of the remaining liquid composition from the polymer layer.

2. Method according to Claim 1, **characterized in that** an at least partly optically transparent support is used.

3. Method according to Claim 1 or 2, **characterized in that** a support with a thickness of at least 5 µm is used.

4. Method according to any of the preceding claims, **characterized in that** a support selected from a plastics support, a glass support or a quartz support is used.

5. Method according to any of the preceding claims, **characterized in that** a photopolymerizable liquid composition which comprises at least one photopolymerizable substance is used.

6. Method according to Claim 5, **characterized in that** monomers selected from acrylic monomers, vinylaromatic monomers, functionalized polyvinylpyrrolidones and any combinations thereof are used.

7. Method according to any of the preceding claims, **characterized in that** a photopolymerizable liquid composition which comprises at least one photoinitiator is used.

8. Method according to any of the preceding claims, **characterized in that** the layer thickness of the polymer is controlled through variation of
(i) the intensity of irradiation,
(ii) the duration of irradiation or/and
(iii) the addition of substances which absorb the polymerization light.

9. Method according to any of the preceding claims, **characterized in that** a polymer layer with a thickness of ≤ 500 µm, in particular of ≤ 5 µm, is produced.

10. Method according to any of the preceding claims, **characterized in that** a polymer layer which comprises at least one indicator is produced.

11. Method according to Claim 10, **characterized in that** a macromolecular substance is used as indicator.

12. Method according to Claim 10 or 11, **characterized in that** a catalytic substance, in particular an enzyme, is used as indicator.

13. Method according to any of the preceding claims, **characterized in that** a crosslinked polymer layer is produced.

14. Method according to Claim 13, **characterized in that** a macromolecule is entrapped in the crosslinked polymer layer.

15. Method according to any of the preceding claims, **characterized in that** the irradiation is carried out with UV light.

16. Method according to any of the preceding claims, **characterized in that** the polymerization is carried out as continuous process.

17. Method according to Claim 16, **characterized in that** the photopolymerizable liquid composition is continuously applied at a first position to a moving support and continuously irradiated at a second position.

18. Device for producing polymer layers, comprising:
(a) means for receiving and where appropriate for transporting a support,
(b) means for applying a photopolymerizable liquid composition to the support and
(c) means for irradiating the photopolymerizable liquid composition through the support in order to form a polymer layer with predetermined thickness on the support, and
(d) where appropriate means for removing unpolymerized liquid composition from the polymer layer.

19. Use of the method according to any of Claims 1 to 17 or of the device according to Claim 18 for producing a sensor, where the polymer layer comprises at least one indicator.

20. Use according to Claim 19, **characterized in that** the indicator is present in the polymer layer in immobilized form.

21. Use according to Claim 19 or 20, **characterized in that** the polymer layer comprises an enzyme, where appropriate in the form of an enzyme-coenzyme complex.

22. Use according to any of Claims 19 to 21, **characterized in that** the sensor is selected from optical or/and electrochemical sensors.

23. Method for producing a sensor, comprising the steps:
(a) provision of a transparent support,
(b) application of a photopolymerizable liquid composition which comprises at least one indicator to the support,
(c) irradiation of the photopolymerizable liquid composition through the support in such a way that a polymer layer with a predetermined thickness is formed on the support,
(d) removal of the remaining liquid composition from the polymer layer and
(e) introduction of the support with the polymer layer comprising the indicator into a sensor which comprises means for detecting the reaction of the indicator with an analyte in a sample.

24. Method according to Claim 23, **characterized in that** the indicator comprises an enzyme, where appropriate in the form of an enzyme-coenzyme complex.

25. Method according to Claim 24, **characterized in that** the detection means comprise optical or/and electrochemical detection means.

26. Method according to Claim 25, **characterized in that** the optical detection means comprise a light source for irradiating the polymer layer and a detector for collecting light from the polymer layer.

27. Method according to Claim 26, **characterized in that** the light source is provided to beam light through the support into the polymer layer.

28. Method according to Claim 26 or 27, **characterized in that** a detector is provided to collect fluorescence emission from the polymer layer.

## Revendications

1. Procédé de fabrication de couches polymères sur un support transparent, comprenant les étapes de :
(a) mise à disposition d'un support,
(b) application d'une composition liquide photopolymérisable sur le support,
(c) irradiation de la composition liquide photopolymérisable à travers le support de telle manière qu'une couche polymère ayant une épaisseur préalablement déterminée est formée sur le support, et
(d) élimination de la composition liquide restante hors de la couche polymère.

2. Procédé selon la revendication 1, **caractérisé en ce que** l'on utilise un support au moins partiellement optiquement transparent.

3. Procédé selon la revendication 1 ou 2, **caractérisé en ce que** l'on utilise un support ayant une épaisseur d'au moins 5 µm.

4. Procédé selon l'une des revendications précédentes, **caractérisé en ce que** l'on utilise un support choisi parmi un support en plastique, un support en verre ou un support en quartz.

5. Procédé selon l'une des revendications précédentes, **caractérisé en ce que** l'on utilise une composition liquide photopolymérisable qui contient au moins une substance photopolymérisable.

6. Procédé selon la revendication 5, **caractérisé en ce que** l'on utilise des monomères choisis parmi des monomères acryliques, des monomères aromatiques vinyliques, des polyvinylpyrrolidones fonctionnalisées et des combinaisons quelconques de ceux-ci.

7. Procédé selon l'une des revendications précédentes, **caractérisé en ce que** l'on utilise une composition liquide photopolymérisable qui contient au moins un photoinitiateur.

8. Procédé selon l'une des revendications précédentes, **caractérisé en ce que** l'on contrôle l'épaisseur de la couche du polymère en variant
(i) l'intensité de l'irradiation
(ii) la durée de l'irradiation ou/et
(iii) l'ajout de substances qui absorbent la lumière de polymérisation.

9. Procédé selon l'une des revendications précédentes, **caractérisé en ce que** l'on fabrique une couche polymère ayant une épaisseur ≤ 500 µm, en particulier ≤ 5 µm.

10. Procédé selon l'une des revendications précédentes, **caractérisé en ce que** l'on fabrique une couche polymère qui contient au moins un indicateur.

11. Procédé selon la revendication 10, **caractérisé en ce que** l'on utilise une substance macromoléculaire comme indicateur.

12. Procédé selon la revendication 10 ou 11, **caractérisé en ce que** l'on utilise une substance catalytique comme indicateur, en particulier une enzyme.

13. Procédé selon l'une des revendications précédentes, **caractérisé en ce que** l'on fabrique une couche polymère réticulée.

14. Procédé selon la revendication 13, **caractérisé en ce que** l'on inclut une macromolécule dans la couche polymère réticulée.

15. Procédé selon l'une des revendications précédentes, **caractérisé en ce que** l'on effectue l'irradiation avec de la lumière UV.

16. Procédé selon l'une des revendications précédentes, **caractérisé en ce que** l'on effectue la polymérisation en procédé continu.

17. Procédé selon la revendication 16, **caractérisé en ce que** la composition liquide photopolymérisable est appliquée continuellement en une première position sur un support se déplaçant et est continuellement irradié en une seconde position.

18. Dispositif de fabrication de couches polymères, comprenant :
(a) un moyen de recueil et le cas échéant de transport d'un support,
(b) un moyen d'application d'une composition liquide photopolymérisable sur le support et
(c) un moyen d'irradiation de la composition liquide photopolymérisable à travers le support pour obtenir la formation sur le support d'une couche polymère ayant une épaisseur préalablement déterminée, et
(d) le cas échéant un moyen d'élimination de la composition liquide n'ayant pas été polymérisée par la couche polymère.

19. Utilisation du procédé selon l'une des revendications 1 à 17 ou du dispositif selon la revendication 18 dans la fabrication d'un capteur, la couche polymère contenant au moins un indicateur.

20. Utilisation selon la revendication 19, **caractérisée en ce que** l'indicateur se trouve sous forme immobilisée dans la couche polymère.

21. Utilisation selon la revendication 19 ou 20, **caractérisée en ce que** la couche polymère contient une enzyme, le cas échéant sous forme d'un complexe d'enzyme-coenzyme.

22. Utilisation selon l'une des revendications 19 à 21, **caractérisée en ce que** le capteur est choisi parmi des capteurs optiques ou/et électrochimiques.

23. Procédé de fabrication d'un capteur, comprenant les étapes :
(a) de mise à disposition d'un support transparent,
(b) d'application d'une composition liquide photopolymérisable, qui contient au moins un indicateur, sur le support,
(c) d'irradiation de la composition liquide photopolymérisable à travers le support de telle manière qu'une couche polymère ayant une épaisseur préalablement déterminée est formée sur le support,
(d) d'élimination de la composition liquide restante hors de la couche polymère
(e) d'introduction du support ayant la couche polymère contenant l'indicateur dans un capteur, qui contient le moyen de preuve de la réaction de l'indicateur avec un analyte dans un échantillon.

24. Procédé selon la revendication 23, **caractérisé en ce que** l'indicateur comprend une enzyme, le cas échéant sous forme d'un complexe d'enzyme-coenzyme.

25. Procédé selon la revendication 24, **caractérisé en ce que** les moyens de preuve comprennent des moyens de preuve optiques ou/et électrochimiques.

26. Procédé selon la revendication 25, **caractérisé en ce que** les moyens de preuve optiques comprennent une source de lumière pour irradier la couche polymère et un détecteur pour recueillir la lumière à la sortie de la couche polymère.

27. Procédé selon la revendication 26, **caractérisé en ce que** la source de lumière est prévue pour irradier la lumière dans la couche polymère à travers le support.

28. Procédé selon la revendication 26 ou 27, **caractérisé en ce qu'**un détecteur est prévu pour recueillir l'émission de fluorescence à la sortie de la couche polymère.
